Europäisches Patentamt

European Patent Office

Office européen des brevets

(19)

(11) Publication number: **0 375 841**
**A1**

(12) # EUROPEAN PATENT APPLICATION

(21) Application number: **89116735.5**

(22) Date of filing: **09.09.89**

(51) Int. Cl.⁵: **C12N 9/60, C12N 15/57**

(30) Priority: **13.09.88 US 243734**

(43) Date of publication of application:
**04.07.90 Bulletin 90/27**

(84) Designated Contracting States:
**AT BE CH DE ES FR GB GR IT LI LU NL SE**

(71) Applicant: **THE GENERAL HOSPITAL
CORPORATION
55 Fruit Street
Boston MA 02114(US)**

(72) Inventor: **Smith, John A.
19 Thatcher Street, No.5
Brookline Massachusetts 02146(US)**
Inventor: **Chang, Yie-Hwa
91 Winslow Avenue
Somerville Massachusetts 02143(US)**

(74) Representative: **Klein, Otto, Dr. et al
Hoechst AG Zentrale Patentabteilung
Postfach 80 03 20
D-6230 Frankfurt am Main 80(DE)**

(54) Isolation, purification, and characterization of the aminopeptidases: AP1 and AP122.

(57) This invention is directed toward substrate-specific aminopeptidase enzymes, and specifically toward a substrate-specific aminopeptidase produced via natural or recombinant methods. The invention is further directed to a method for purifying and using the substrate-specific aminopeptidase.

EP 0 375 841 A1

## ISOLATION, PURIFICATION, AND CHARACTERIZATION OF THE AMINOPEPTIDASES: AP1 AND AP122

Field of the Invention

The invention is directed to the isolation and purification of substrate-specific aminopeptidases and to the enzyme themselves.

Background of the Invention

Enzymes which are capable of cleaving a peptide bond between the amino-terminal residue of a protein or peptide and an adjacent amino acid residue are known as "aminopeptidases." Such enzymes have been identified in a variety of microorganisms, such as E. coli, Bacillus licheniformis, Bacillus subtilus, Salmonella typhimurium and Mycoplasma salivarium (Carter, T.H. et al., J. Bacteriol. 159:453-459 (1984); Hayashi, S., J. Biol. Chem. 259:10448-10454 (1984); Hermsdorf, C.L., Biochem. 17:3370-3376 (1978); McHugh, G.L. et al., J. Bacteriol 120:364-371 (1974); Miller, C.G. et al., J. Bacteriol. 135:603-611 (1978), Simmonds, S., Biochem. 9:1-8 (1970); and Shibata, K. et al., J. Bacteriol. 169:3409-3413 (1987)).

One example of an aminopeptidase is the leucine-specific aminopeptidase produced in mammalian intestinal mucosa and kidneys. The leucine-specific aminopeptidase of swine intestinal fluid has been purified and characterized, and found to contain a $Zn^{+2}$ ion. The enzyme has a broad substrate specificity with respect to the N- terminal residue of the substrate peptide. By successive hydrolysis of N-terminal peptide bonds, it can degrade peptides to free amino acids. The enzyme has been found to be activated by $Mn^{+2}$, which is capable of replacing the zinc ion (White, A. et al., In: Principles of Biochemistry, 5th Ed., McGraw Hill, NY, NY (1973)). A second example of an aminopeptidase is the methionine-specific aminopeptidase of E. coli (Ben-Bassat et al., J. Bacteriol. 169:751-757 (1987)). A theoretical discussion of the properties of aminopeptidases is provides by Sherman, F. et al. (BioEssays 3:27-31 (1985)).

Yeast, and in particular, the yeast Saccharomyces cerevisiae, have also been found to contain aminopeptidases. Trumbly, R.J. et al. (J. Bacteriol. 156:36-48 (1983)), for example, identified a yeast leucine aminopeptidase which was capable of cleaving a leucine-containing peptide. Yeast peptidases are reviewed by Achstetter, T. et al. (Yeast 1:139-157 (1985)), which reference is herein incorporated by reference. Aminopeptidases are also produced in the human small intestine (Martin, D.W. et al., In: Harper's Review of Biochemistry, 19th Ed., Lange Medical Publications, Los Altos, CA (1983)).

Aminopeptidases are important in the processing and degradation of peptides and proteins. They have been used as an alternative to Edman degradation in the determination of the amino acid sequence of polypeptides and proteins.

Substrate-specific aminopeptidases can also be used to specifically cleave amino acid residues from the amino-terminus of a protein or peptide. Thus, such enzymes may have value in removing signal sequences, nuclear localization sequences, leader sequences, secretion sequences etc., which may be present at the amino-terminal end of a peptide or protein. Since many proteins, produced through recombinant DNA technology, or by other means contain such sequences, aminopeptidases can be used to convert such molecules into their mature and/or functional forms. The identification and availability of additional substrate-specific aminopeptidases is therefore highly desirable.

Summary of the Invention

According to the present invention, substrate-specific aminopeptidases, capable of cleaving the peptide bond between the carboxyl group of a particular amino-terminal amino acid residue and the amino group of an adjacent amino acid residue were isolated and substantially purified. The present invention therefore relates to the purification of these enzymes, to the uses for the enzymes, and to the substantially purified enzymes themselves.

In detail, the invention concerns a substantially purified substrate-specific aminopeptidase enzyme having an ability to cleave the peptide bond between an amino-terminal methionine residue, and a penultimate amino-terminal amino acid residue selected from the group consisting of: Val, Ala, Arg, Cys, Gly, His, Ile, Leu, Lys, Met and Phe.

The invention also concerns a substantially purified substrate-specific aminopeptidase enzyme having an ability to cleave the peptide bond between an amino-terminal residue X, and an adjacent Ile residue of a peptide, wherein X is an amino acid residue selected from the group consisting of: Arg, Gln, Leu, Met, Phe and Trp.

The invention also concerns a substantially purified substrate-specific aminopeptidase enzyme having an ability to cleave the peptide bond between an amino-terminal methionine residue, and a penultimate amino-terminal amino acid residue selected from the group consisting of: Arg, Cys, Gly, His, Ile, Leu, Lys, Met, Phe, Ser or Thr.

The invention also concerns a substantially purified substrate-specific aminopeptidase enzyme having an ability to cleave the peptide bond between an amino-terminal residue X, and an adjacent Ile residue of a peptide, wherein X is an amino acid residue selected from the group consisting of: Val, Ala, Arg, Gln, His, Ile, Leu, Met, Phy, Pro, Ser, Thr and Trp.

The invention also concerns the above-described substrate-specific aminopeptidases which are AP1 or AP122.

The invention also concerns a recombinant DNA molecule comprising a genetic sequence coding for any of the above-described substrate-specific aminopeptidases.

The invention also concerns any of the above-described substrate-specific aminopeptidases which is produced by the expression of a recombinant molecule.

The invention also concerns a method for recovering the above-described substantially purified substrate-specific aminopeptidase AP1 from a sample containing the aminopeptidase comprising the following steps:

(a) recovering crude substrate-specific aminopeptidase from a sample containing the aminopeptidase;

(b) subjecting the crude substrate-specific aminopeptidase from step (a) to serial ion exchange chromatography on CM-Sepharose and DEAE-Sepharose CL-6B; and

(c) recovering any substrate-specific aminopeptidase purified by ammonium sulfate precipitation from the eluate from the serial ion exchange chromatography.

The invention further pertains to the above-described method which additionally comprises the steps of:

(d) subjecting the recovered substrate-specific aminopeptidase of step (c) to Phenyl-Sepharose hydrophobic interaction chromatography; and

(e) recovering any substrate-specific aminopeptidase purified by the Phenyl-Sepharose hydrophobic interaction chromatography.

The invention further pertains to the above-described method which additionally comprises the steps of:

(f) subjecting the recovered substrate-specific aminopeptidase of step (e) to Sepharose CL-6B chromatography; and

(g) recovering any substrate-specific aminopeptidase purified by the Sepharose CL-6B chromatography.

The invention also concerns a method for recovering the above-described substantially purified substrate-specific aminopeptidase AP122 from a sample containing the aminopeptidase comprising the following steps:

(a) recovering crude substrate-specific aminopeptidase from a sample containing the aminopeptidase;

(b) subjecting the crude substrate-specific aminopeptidase from step (a) to ion exchange chromatography; and

(c) recovering any substrate-specific aminopeptidase purified by ammonium sulfate precipitation from the eluate from the ion exchange chromatography.

The invention further pertains to the above-described method which additionally comprises the steps of:

(d) subjecting the recovered substrate-specific aminopeptidase of step (c) to Phenyl-Sepharose hydrophobic interaction chromatography; and

(e) recovering any substrate-specific aminopeptidase purified by the Phenyl-Sepharose hydrophobic interaction chromatography.

The invention further pertains to the above-described method which additionally comprises the steps of:

(f) subjecting the recovered substrate-specific aminopeptidase of step (e) to hydroxylapatite chromatography; and

(g) recovering any substrate-specific aminopeptidase purified by the hydroxylapatite chromatography.

The invention additionally provides a method for removing an amino-terminal methionine residue from a peptide containing the amino-terminal residue, and containing as a penultimate amino-terminal amino acid residue, an amino acid selected from the group consisting of: Val, Ala, Arg, Cys, Gly, His, Ile, Leu, Lys, Met and Phe, which method comprises incubating the peptide in the presence of the substrate-specific aminopeptidase AP1 to thereby remove the amino-terminal residue.

The invention additionally provides a method for removing an amino-terminal methionine residue from a peptide containing the amino-terminal residue, and containing as a penultimate amino-terminal amino acid residue, an amino acid selected from the group consisting of Arg, Cys, Gly, His, Ile, Leu, Lys, Met, Phe, Ser and Thr, which method comprises incubating the peptide in the presence of the substrate-specific aminopeptidase AP122 to thereby remove the amino-terminal residue.

The invention additionally provides a method for removing an amino-terminal residue X from a peptide containing the terminal residue X, and an adjacent Ile residue, wherein X is selected from the group consisting of Arg, Gln, Leu, Met, Phe and Trp, which method comprises incubating the peptide in the presence of the substrate-specific aminopeptidase AP1 to thereby remove the amino-terminal residue.

The invention additionally provides a method for removing an amino-terminal residue X from a peptide containing the terminal residue X, and an adjacent Ile residue, wherein X is selected from the group consisting of Val, Ala, Arg, Gln, His, Ile, Leu, Met, Phe, Pro, Ser, Thr and Trp, which method comprises incubating the peptide in the presence of the substrate-specific aminopeptidase AP122 to thereby remove the amino-terminal residue.

The invention additionally concerns the embodiment of the above methods wherein the substrate-specific aminopeptidase is immobilized to a solid support.


Detailed Description of the Invention


I. Isolation and Purification of the Substrate-Specific Aminopeptidases AP1 and AP122 of the Present Invention

In accordance with the present invention, either of the substrate-specific aminopeptidases of the present invention can be isolated from a sample containing the enzyme. Substrate-specific aminopeptidases appear to be ubiquitous, being found in all eukaryotes, including plants, animals, and multicellular organisms. A different enzyme having methionine-specific aminopeptidase activity is also believed to be present in prokaryotes. Therefore, any cell source is contemplated in this invention. As used herein, the sample containing either of the enzymes will be referred to simply as "sample." Unless specified as being unique to one or the other of the substrate-specific aminopeptidases of the present invention, the recombinant DNA methods described herein can be employed to clone and express either or both of the aminopeptidases of the present invention.

In the following description of the purification of the substrate-specific aminopeptidases of the present invention, the activity of the aminopeptidases may be assayed by any convenient method. The preferred assay for substrate-specific aminopeptidase activity is as follows:

Enzyme activity can be assayed using either Met-pNA (methionine para-nitroanilide) or the peptide, Met-Ala-Ser, as the substrate. When Met-pNA is used as the substrate, an aliquot of 10 $\mu$l from each fraction collected from a chromatographic column is added to a labeled well of a microtiter plate containing 45 $\mu$l of a solution which contains 4 mM Met-pNA, 10 mM HEPES-K$^+$, pH 7.35, 0.2 mM CoCl$_2$, 0.2 M KCl, 1.5 mM MgCl$_2$, 0.02% NaN$_3$. All of the reactions are preferably conducted using a microtiter plate. After incubation for one hour at room temperature, the absorbance of the sample at 414 nm is determined using a plate reader.

When the assay is conducted using Met-Ala-Ser, as the substrate an aliquot of 10 $\mu$l from each fraction collected from a chromatographic column is added to a labeled well of a microtiter plate containing 45 $\mu$l of the solution which contains 4 mM Met-Ala-Ser, 10 mM HEPES-K$^+$, pH 7.35, 0.2 mM CoCl$_2$, 0.2 M KCl, 1.5 mM MgCl$_2$, 0.02% NaN$_3$, 10 $\mu$g of L-amino acid oxidase, 115 $\mu$g of horseradish peroxidase and 10 $\mu$g of o-dianisidine dihydrochloride. The reactions are carried out at room temperature, for one hour, and the adsorbance at 450 nm, or equivalently at 414 nm, is recorded.

The isolation and purification of the substrate-specific aminopeptidases of the present invention will hereinafter be described from a yeast sample, although its is to be understood that other samples could be used as the source material.

Yeast cells, preferably Saccharomyces cerevisiae, are grown in suitable medium (preferably YPD medium: 1% yeast extract, 2% Bactopeptone, 2% glucose) to an A$_{600}$ of 5-20. Preparations of cells harvested at an A$_{600}$ of 8, or at an A$_{600}$ of 18-20 are found to have equivalent substrate-specific aminopeptidase activity. Cells are harvested, preferably by centrifugation, and resuspended in YPD medium containing 15% glycerol. The cells may then be frozen to -70 $^{\circ}$C, and stored for an extended period.

Frozen cells are thawed, preferably by incubation in a 30 °C water bath, and collected by centrifugation at room temperature. The pelleted cells are resuspended in a suitable buffer such as Buffer S (1 M sorbitol, 50 mM Tris, pH 7.8, 10 mM $MgCl_2$, 3 mM DTT), supplemented with lyticase, and incubated at 30 °C, to permit spheroplast formation. The spheroplasts are then collected, washed in additional Buffer S, lysed and homogenized in hypotonic buffer, such as buffer A (10 mM HEPES-K$^+$, pH 7.0, 1.5 mM $MgCl_2$, 10 mM KCl, and 0.5 mM DTT). Homogenization is preferably accomplished at 4 °C using a Dounce homogenizer. Yeast substrate-specific aminopeptidase is released into the cellular lysate by gentle shaking. After removing any debris by centrifugation, glycerol is added to the supernatant of the lysate (10% final concentration) in order to increase the stability of the substrate-specific aminopeptidase.

The above-described supernatant of the cellular lysate is then concentrated to 100 ml by the use of a PM-10 ultrafiltration membrane and dialyzed three times against 2 liters of a suitable buffer, such as Buffer H (10 mM HEPES-K$^+$, pH 7.35, 1.5 mM $MgCl_2$, 0.5 mM DTT, 0.2% $NaN_3$, 10% glycerol), supplemented to contain 0.05 M KCl. Residual cell biomaterials in the supernatant are removed to avoid protein-biomaterial aggregation in subsequent steps. Ion exchange chromatography can be used for this procedure. In this step, the ion exchange used is preferably CM-Sepharose chromatography, most preferably employing CM-Sepharose CL-6B. The concentrated supernatant is preferably loaded on a CM-Sepharose CL-6B column which has been connected to a DEAE-Sepharose CL-6B column such that the eluate from the CM-Sepharose CL-6B column is applied to the DEAE-Sepharose CL-6B column continually. Both columns are equilibrated against the same buffer as that used for dialysis. The two connected columns are then eluted with 2 liters of buffer H (supplemented to contain 0.05 M KCl), or another suitable buffer. The eluate is collected.

Solid ammonium sulfate is added to the eluate to make a 50% saturated ammonium sulfate solution at 4 °C. This solution is stirred until a precipitate forms, or, preferably, overnight at 4 °C. The precipitate is removed by centrifugation, preferably at 10,000 rpm for 30 min. Solid ammonium sulfate is then added to the supernatant to make an 80% saturated ammonium sulfate solution. This solution is stirred until a precipitate forms, or, preferably, overnight at 4 °C. The precipitate is collected by centrifugation at 10,000 rpm for 30 min at 4 °C. This precipitate is redissolved in a minimum amount of buffer H containing 0.2 M KCl, to form a solution containing both aminopeptidase AP1 and AP122.

Aminopeptidases AP1 and AP122 may be further purified using hydrophobic interaction chromatography (preferably using Phenyl-Sepharose CL-4B chromatography). The redissolved precipitate is dialyzed three times against 1 liter of buffer H, or another suitable buffer, supplemented to contain 0.2 M KCl. An equal volume of 80% saturated ammonium sulfate solution, 10 mM HEPES-K$^+$, pH 7.35, 10% glycerol, 0.02% $NaN_3$ is then added to the solution containing the aminopeptidase. This mixture is then applied to a Phenyl-Sepharose CL-4B column equilibrated with 40% saturated ammonium sulfate solution, 10 mM HEPES-K$^+$, pH 7.35, 10% glycerol, 0.25 mM DTT, 0.1 M KCl, 0.02% $NaN_3$. The column is first eluted with the same buffer as that described above and then with a linear gradient of 40% to 0% ammonium sulfate solution, 10 mM HEPES-K$^+$, pH 7.35, 10% glycerol, 0.25 mM DTT, 0.02% $NaN_3$, 100 mM KCl at 79 ml/hr. Fractions (typically of approximately 4 ml) are collected. The fractions are numbered consecutively with the first collected fraction being designated as "fraction 1." Fractions having a conductivity of approximately 67.5 ms (approximately fractions 45-53) contain the aminopetidase AP1. Fractions having a conductivity of approximately 35 ms (approximately fractions 81-93) contain the aminopetidase AP122. The aminopeptidases are further purified using either hydroxylapatite chromatography (for AP122), or Sepharose CL-6B chromatography (for AP1).

The pooled fractions containing AP122 aminopeptidase activity from the Phenyl-Sepharose CL-4B chromatography are concentrated to 5 ml by the use of a PM-10 ultrafiltration membrane. The concentrated pooled fraction is dialyzed three times against 1 liter of 0.01 M potassium buffer, 0.5 mM DTT, 1.5 mM $MgCl_2$, 0.2% $NaN_3$, 5 mM KCl, 10% glycerol, and applied to a hydroxylapatite column (2.5 x 20 cm) equilibrated with the same buffer as that used for dialysis. The column is eluted with a linear gradient of 0.01 M to 0.5 M potassium phosphate buffer 0.5 mM DTT, 0.2% $NaN_3$, 5 mM KCl, 10% glycerol at approximately 19 ml/hr. Fractions of approximately 4 ml are collected, and assayed for aminopeptidase activity as described above. Typically, AP122 aminopeptidase is found at a conductivity of approximately 9.1 ms (approximately fractions 93-101). The fractions containing AP122 aminopeptidase activity are pooled.

The pooled fractions from the Phenyl-Sepharose CL-4B chromatography having AP1 aminopeptidase activity are concentrated to a volume of 2 ml using a PM-10 ultrafiltration membrane. The solution is then dialyzed three times against 1 liter of buffer H (supplemented to contain 0.2 M KCl) and applied to a Sepharose CL-6B column equilibrated with the same buffer used for dialysis. The column is eluted with buffer H containing 0.2 M KCl at 18.5 ml/hr. Fractions of approximately 4 ml are collected, and assayed for aminopeptidase activity as described above. Typically, AP1 aminopeptidase is found at a peak of $A_{414}$ adsorbance in the final third of the elution (approximately fractions 53-59 of 77 total fractions). The fractions containing AP1 aminopeptidase activity are pooled.

Using this series of chromatography steps, yeast substratespecific aminopeptidase AP1 was purified to near homogeneity. The enzyme has an apparent molecular weight of 81,000.

The terms peptide, polypeptide, and protein are all intended to refer to polymers of amino acid residues. A peptide is a polymer of at least two amino acid residues joined to one another by a peptide bond. A polypeptide is a polymer of several amino acid residues joined to one another by a peptide bond. A protein is a large polymer of many amino acid residues joined to one another by a peptide bond.

The term "aminopeptidase" as used herein is intended to refer to an enzyme capable of attacking and cleaving the peptide bond which joins the amino-terminal amino acid residue of a peptide (and thus also of a polypeptide or protein) to the penultimate amino-terminal amino acid residue of that peptide (or polypeptide or protein).

The aminopeptidases of the present invention are "substrate-specific aminopeptidases." An aminopeptidase is said to be "substrate-specific" if only certain peptides or proteins (i.e. not every peptide or protein) can serve as a substrate for the enzyme. Such suitable substrates possess particular amino-terminal and penultimate amino-terminal residues, which are joined by a peptide bond. The substrate specificity of the aminopeptidase is defined by its ability or inability to cleave a particular substrate. The substrate specificity of the aminopeptidases of the present invention is defined below.

The present invention concerns a substrate-specific aminopeptidase enzyme which is "substantially pure" or which as been "substantially purified." As used herein, the terms "substantially pure" or "substantially purified" are intended to be equivalent, and to describe a substrate-specific aminopeptidase which is substantially free of a compound normally associated with the enzyme in its natural state, i.e., a protein, carbohydrate, lipid, etc. The term is further meant to describe a substrate-specific aminopeptidase which is homogeneous by one or more of the assays of purity or homogeneity used by those of skill in the art. For example, a substantially pure substrate-specific aminopeptidase will show constant and reproducible characteristics within standard experimental deviations for parameters such as the following: molecular weight, chromatographic techniques, etc. The term, however, its not meant to exclude artificial or synthetic mixtures of the enzymes with other compounds. The term is also not means to exclude the presence of impurities which do not interfere with the biological activity of the enzyme, and which may be present, for example, due to incomplete purification.


II. Characterization of the Substrate-Specific Aminopeptidases of the Present Invention


Molecular Weight of AP1

SDS-polyacrylamide gels of the purified sample reveal that AP1 migrates as a single band of about apparent molecular weight 81,000 when stained with Coomassie blue. The electrophoresis was performed according to the method of Laemmli, U.K. (Nature 227: 680-685 (1970)) using a 10% gel. The gel was stained with Coomassie blue. Standard proteins of 45, 66, 97, 116, and 205 kd molecular weight were used to calibrate the gel. The apparent molecular weight of AP122 has not been determined.


Substrate-Specificity.

A partial determination of substrate-specificity of AP1 and AP122 was carried out using a set of thirty six pure peptides. The peptides had either of two sequences:

(I)     MET-X-ILE-PRO-GLU-THR
or (II)     X-ILE-PRO-GLU-THR
wherein "X" refers to one of the following eighteen amino acids: Val, Ala, Arg, Asp, Cys, Glu, Gln, Gly, His, Ile, Leu, Lys, Met, Phe, Pro, Ser, Thr and Trp.

AP1 was found to be able to cleave those peptides of group I wherein "X" was either Val, Ala, Arg, Cys, Gly, His, Ile, Leu, Lys, Met or Phe. No other tested peptide of group I could be cleaved by AP1. AP1 was able to cleave only those peptides of group II wherein "X" was Arg, Gln, Leu, Met, Phe or Trp. No other tested peptides of group II could be cleaved.

AP122 was found to be able to cleave those peptides of group I wherein "X" was either Arg, Cys, Gly, His, Ile, Leu, Lys, Met, Phe, Ser or Thr. No other tested peptide of group I could be cleaved. AP122 was able to cleave those peptides of group II wherein "X" was either Val, Ala, Arg, Gln, His, Ile, Leu, Met, Phe, Pro, Ser, Thr or Trp. No other peptide of group II tested could be cleaved.

III. Genetic Engineering of the Aminopeptidases of the Present Invention

The identification of fractions which contain substantially purified AP1 or AP122 activity permits the amino acid sequence of these enzymes to be determined, and further permits these molecules to be produced through the application of recombinant DNA techniques. Thus, the present invention includes not only substantially purified AP1 and AP122, and methods for their use, but also includes the amino acid sequences of these enzymes, the genetic sequences coding for these enzymes, vehicles containing such genetic sequences, hosts transformed therewith, enzyme production by transformed host expression, and utilization of the enzymes in the cleavage of substrate-containing peptides or proteins. AP1 is the preferred substrate-specific aminopeptidase of the present invention.

In order to obtain the amino acid sequence of the enzymes, the enzymes in the substantially purified fractions are recovered as by electroelution from a polyacrylamide gel. The recovered molecules may then be sequenced, preferably using an automated sequenator, and the amino acid sequence of the enzyme thereby determined.

Alternatively, and more preferably, the amino acid sequence of the enzymes is determined through an analysis of the DNA or, more preferably, the cDNA sequence, of the gene which encodes the enzyme. In order to obtain these nucleic acid sequences, a source which contains the AP1 or AP122 gene or cDNA sequence is screened with oligonucleotide probes which encode fragments of the AP1 or AP122 enzyme.

In order to prepare the oligonucleotide probes, the substantially purified enzyme is recovered and fragmented as with cyanogen bromide, or with proteases such as papain, chymotrypsin, trypsin, etc. (Oike, Y., et al., J. Biol. Chem. 257:9751-9758 (1982); Liu, C., et al., Int. J. Pept. Protein Res. 21:209-215 (1983)). The resulting peptides are separated, preferably by reverse-phase HPLC, and subjected to amino acid sequencing. To accomplish this task, the peptides are preferably analyzed by automated sequenators.

Once one or more suitable peptide fragments have been sequenced, the DNA sequences capable of encoding them are examined. If the peptides are greater than 10 amino acids long, this sequence information is generally sufficient to permit one to clone a gene sequence such as those which encode the enzymes of the present invention. Because the genetic code is degenerate, however, more than one codon may be used to encode a particular amino acid (Watson, J.D., In: Molecular Biology of the Gene, 3rd Ed., W.A. Benjamin, Inc., Menlo Park, CA (1977), pp. 356-357). Using the genetic code, one or more different oligonucleotides can be identified, each of which would be capable of encoding the enzyme peptides. The probability that a particular oligonucleotide will, in fact, constitute the actual enzyme fragment-encoding sequence can be estimated by considering abnormal base pairing relationships and the frequency with which a particular codon is actually used (to encode a particular amino acid) in eukaryotic cells. Such "codon usage rules" are disclosed by Lathe, R., et al., J. Molec. Biol. 183:1-12 (1985). Using the "codon usage rules" of Lathe, a single oligonucleotide, or a set of oligonucleotides, that contains a theoretical "most probable" nucleotide sequence capable of encoding the enzyme fragment's peptide sequences is identified.

Techniques for synthesizing oligonucleotides are disclosed by, for example, Wu, R., et al., Prog. Nucl. Acid. Res. Molec. Biol. 21:101-141 (1978)). Procedures for constructing recombinant molecules in accordance with the above-described method are disclosed by Maniatis, T., et al., In: Molecular Cloning, A Laboratory Manual, Cold Spring Harbor Press, Cold Spring Harbor, NY (1984), which reference is herein incorporated by reference.

Although occasionally an amino acid sequence may be encoded by only a single oligonucleotide, frequently the amino acid sequence may be encoded by any of a set of similar oligonucleotides. Importantly, whereas all of the members of this set contain oligonucleotides which are capable of encoding the peptide fragment and, thus, potentially contain the same oligonucleotide sequence as the gene which encodes the peptide fragment, only one member of the set contains the nucleotide sequence that is identical to the nucleotide sequence of the gene. Because this member is present within the set, and is capable of hybridizing to DNA even in the presence of the other members of the set, it is possible to employ the unfractionated set of oligonucleotides in the same manner in which one would employ a single oligonucleotide to clone the gene that encodes the peptide.

The oligonucleotide, or set of oligonucleotides, containing the theoretical "most probable" sequence capable of encoding the enzyme fragment peptide is used to identify the sequence of a complementary oligonucleotide or set of oligonucleotides which is capable of hybridizing to the "most probable" sequence, or set of sequences. An oligonucleotide containing such a complementary sequence can be employed as a probe to identify and isolate a gene sequence which encodes the enzyme (Maniatis, T., et al., Molecular Cloning, A Laboratory Manual, Cold Spring Harbor Press, Cold Spring Harbor, NY (1982)).

The DNA probe may be labeled with a detectable group. Such detectable group can be any material having a detectable physical or chemical property. Such materials have been well-developed in the field of immunoassays and in general most any label useful in such methods can be applied to the present invention. Particularly useful are enzymatically active groups, such as enzymes (see Clin. Chem. 22:1243 (1976)), enzyme substrates (see British Pat. Spec. 1,548,741), coenzymes (see U.S. Pat. Nos. 4,230,797 and 4,238,565) and enzyme inhibitors (see U.S. Pat. No. 4,134,792); fluorescers (see Clin. Chem. 25:353 (1979)); chromophores; luminescers such as chemiluminescers and bioluminescers (see Clin. Chem. 25:512 (1979)); specifically bindable ligands; proximal interacting pairs; and radioisotopes such as $^3H$, $^{35}S$, $^{32}P$, $^{125}I$ and $^{14}C$. Such labels and labeling pairs are detected on the basis of their own physical properties (e.g., fluorescers, chromophores and radioisotopes) or their reactive or binding properties (e.g., enzymes, substrates, coenzymes and inhibitors). For example, a cofactor-labeled probe can be detected by adding the enzyme for which the label is a cofactor and a substrate for the enzyme. For example, one can use an enzyme which acts upon a substrate to generate a product with a measurable physical property. Examples of the latter include, but are not limited to, beta-galactosidase, alkaline phosphatase and peroxidase.

A suitable oligonucleotide, or set of oligonucleotides, which is capable of encoding a fragment of the gene sequence which encodes the enzyme (or which is complementary to such an oligonucleotide, or set of oligonucleotides) is identified (using the above-described procedure), synthesized, and hybridized by means well known in the art, against a DNA or, more preferably, a CDNA preparation derived from cells which are capable of expressing the enzyme. Single-stranded oligonucleotide molecules complementary to the "most probable" enzyme peptide encoding sequences can be synthesized using procedures which are well known to those of ordinary skill in the art (Belagaje, R., et al., J. Biol. Chem. 254:5765-5780 (1979); Maniatis, T., et al., In: Molecular Mechanisms in the Control of Gene Expression, Nierlich, D.P., et al., Eds., Acad. Press, NY (1976); Wu, R., et al., Prog. Nucl. Acid Res. Molec. Biol. 21:101-141 (1978); Khorana, R.G., Science 203:614-625 (1979)). Additionally, DNA synthesis may be achieved through the use of automated synthesizers. Techniques of nucleic acid hybridization are disclosed by Maniatis, T., et al. (In: Molecular Cloning, A Laboratory Manual, Cold Spring Harbor Laboratories, Cold Spring Harbor, NY (1982)), and by Haymes, B.D., et al. (In: Nucleic Acid Hybridization, A Practical Approach, IRL Press, Washington, DC (1985)), which references are herein incorporated by reference.

A DNA sequence coding for AP1 or AP122 may be derived from a variety of sources. For example, mRNA coding for either enzyme may be isolated from the tissues of any species that produces the enzyme, and identified, using the Northern blot method (Alwine et al., Method Enzymol. 68:220-242 (1979)) and labeled oligonucleotide probes. The mRNA may then be converted to cDNA by techniques known to those skilled in the art. Alternatively, genomic DNA may be isolated and employed. The source of DNA or cDNA used will preferably have been enriched for the gene sequence which encodes the enzyme. Such enrichment can mostly easily be obtained from cDNA obtained by extracting RNA from cells, such as yeast cells, which produce high levels of the enzyme. An example of such a cell is a Saccharomyces cerevisiae cell.

Any of a variety of methods may be used to clone a gene sequence which encodes the enzymes of the present invention. One such method entails analyzing a shuttle vector library of cDNA inserts (derived from a cell that expresses the enzymes of the present invention) for the presence of an insert which contains a gene sequence which is capable of encoding the enzyme. Such an analysis may be conducted by transfecting cells with the vector, and then assaying for enzyme expression.

To identify and clone the gene sequence capable of encoding either of the enzymes of the present invention, a DNA, or more preferably a cDNA, library is screened for its ability to hybridize with the oligonucleotide probes described above. Suitable DNA preparations (such as human genomic DNA) are enzymatically cleaved, or randomly sheared, and ligated into recombinant vectors. The ability of these recombinant vectors to hybridize to the above-described oligonucleotide probes is then measured. Vectors found capable of such hybridization are then analyzed to determine the extent and nature of the enzyme sequences which they contain. Based purely on statistical considerations, a gene sequence capable of encoding any of the enzymes of the present invention could be unambiguously identified (via hybridization screening) using an oligonucleotide probe having only 18 nucleotides.

In an alternative way of cloning a gene sequence which encodes the enzymes of the present invention, a library of expression vectors is prepared by cloning DNA or, more preferably, cDNA (from a cell capable of expressing the enzyme) into an expression vector. The library is then screened for members capable of expressing a protein which binds to enzyme-specific antibody, and which has a nucleotide sequence that is capable of encoding polypeptides that have the same amino acid sequence as the enzyme, or fragments thereof. In this embodiment, DNA, or more preferably cDNA, is extracted and purified from a cell which is capable of expressing the enzyme. The purified cDNA is fragmented (by shearing, endonuclease digestion, etc.) to produce a pool of DNA or cDNA fragments. DNA or cDNA fragments from this pool are then cloned into an expression vector in order to produce a genomic or cDNA library of expression vectors whose members each contain a unique cloned DNA or cDNA fragment.

Thus, in summary, the identification of the substrate-specific aminopeptidases of the present invention permits the identification of a theoretical "most probable" DNA sequence, or a set of such sequences, capable of encoding peptide sequence of these enzymes. By constructing an oligonucleotide complementary to this theoretical sequence (or by constructing a set of oligonucleotides complementary to the set of "most probable" oligonucleotides), one obtains a DNA molecule (or set of DNA molecules), capable of functioning as a probe to identify and isolate a gene sequence capable of encoding either of the enzymes of the present invention.

Techniques such as, or similar to, those described above have successfully enabled the cloning of genes for human aldehyde dehydrogenases (Hsu, L.C., et al., Proc. Natl. Acad. Sci. USA 82:3771-3775 (1985)), fibronectin (Suzuki, S., et al., Eur. Mol. Biol. Organ. J. 4:2519-2524 (1985)), the human estrogen receptor gene (Walter, P., et al., Proc. Natl. Acad. Sci. USA 82:7889-7893 (1985)), tissue-type plasminogen activator (Pennica, D., et al., Nature 301:214-221 (1983)) and human term placental alkaline phosphatase complementary DNA (Kam, W., et al., Proc. Natl. Acad. Sci. USA 82:8715-8719 (1985)).

## IV. Expression of the Substrate-Specific Aminopeptidase Encoding Sequences

DNA or cDNA molecules which encode the AP1 or AP122 enzyme can be operably linked into an expression vector and introduced into a host cell to enable the expression of the AP1 or AP122 enzyme by that cell. Two DNA sequences (such as a promoter region sequence and a desired enzyme encoding sequence) are said to be operably linked if the nature of the linkage between the two DNA sequences does not (1) result in the introduction of a frame-shift mutation, (2) interfere with the ability of the promoter region sequence to direct the transcription of the desired enzyme encoding gene sequence, or (3) interfere with the ability of the desired enzyme gene sequence to be transcribed by the promoter region sequence.

A DNA sequence encoding AP1 or AP122 may be recombined with vector DNA in accordance with conventional techniques, including blunt-ended or stagger-ended termini for location, restriction enzyme digestion to provide appropriate termini, filling in of cohesive ends as appropriate, alkaline phosphatase treatment to avoid undesirable joining, and ligation with appropriate ligases.

The present invention encompasses the expression of the desired enzyme in either prokaryotic or eukaryotic cells. Preferred eukaryotic hosts include yeast, fungi (especially Aspergillus), mammalian cells (such as, for example, human or primate cells) either in vivo, or in tissue culture.

Yeast are the preferred hosts of the present invention. The use of yeast provides substantial advantages in that yeast can also carry out post-translational peptide modifications including glycosylation. A number of recombinant DNA strategies exist which utilize strong promoter sequences and high copy number of plasmids which can be utilized for production of the desired proteins in yeast. Yeast recognize leader sequences on cloned mammalian gene products and secrete peptides bearing leader sequences (i.e., pre-peptides). Mammalian cells provide post-translational modifications to protein molecules including correct folding or glycosylation at correct sites.

Mammalian cells which may be useful as hosts include cells of fibroblast origin such as VERO or CHO-K1, and their derivatives. For a mammalian host, several possible vector systems are available for the expression of the desired enzyme. A wide variety of transcriptional and translational regulatory sequences may be employed, depending upon the nature of the host. The transcriptional and translational regulatory signals may be derived from viral sources, such as adenovirus, bovine papilloma virus, simian virus, or the like, where the regulatory signals are associated with a particular gene which has a high level of expression. Alternatively, promoters from mammalian expression products, such as actin, collagen, myosin, etc., may be employed. Transcriptional initiation regulatory signals may be selected which allow for repression or activation, so that expression of the genes can be modulated. Of interest are regulatory signals which are temperature-sensitive so that by varying the temperature, expression can be repressed or initiated, or are subject to chemical regulation, e.g., metabolite.

The expression of the desired enzyme in eukaryotic hosts requires the use of eukaryotic regulatory regions. Such regions will, in general, include a promoter region sufficient to direct the initiation of RNA synthesis. Preferred eukaryotic promoters include the promoter of the mouse metallothionein I gene (Hamer, D., et al., J. Mol. Appl. Gen. 1:273-288 (1982)); the TK promoter of Herpes virus (McKnight, S., Cell 31:355-365 (1982)); the SV40 early promoter (Benoist, C., et al., Nature (London) 290:304-310 (1981)); the yeast ga14 gene promoter (Johnston, S.A., et al., Proc. Natl. Acad. Sci. (USA) 79:6971-6975 (1982); Silver, P.A., et al., Proc. Natl. Acad. Sci. (USA) 81:5951-5955 (1984)).

As is widely known, translation of eukaryotic mRNA is initiated at the codon which encodes the first methionine. For this reason, it is preferable to ensure that the linkage between a eukaryotic promoter and a DNA sequence which encodes the desired enzyme (or a functional derivative thereof) does not contain any intervening codons which are capable of encoding a methionine (i.e., AUG). The presence of such codons results either in the formation of a fusion protein (if the AUG codon is in the same reading frame as the desired enzyme encoding DNA sequence) or a frame-shift mutation (if the AUG codon is not in the same reading frame as the desired enzyme encoding sequence).

Preferred prokaryotic hosts include bacteria such as E. coli, Bacillus, Streptomyces, Pseudomonas, Salmonella, Serratia, etc. The most preferred prokaryotic host is E. coli. Bacterial hosts of particular interest include E. coli K12 strain 294 (ATCC 31446), E. coli X1776 (ATCC 31537), E. coli W3110 (F⁻, lambda⁻, prototrophic (ATCC 27325)), and other enterobacteria (such as Salmonella typhimurium or Serratia marcescens, and various Pseudomonas species. The prokaryotic host must be compatible with the replicon and control sequences in the expression plasmid.

To express the desired enzyme (or a functional derivative thereof) in a prokaryotic cell (such as, for example, E. coli, B. subtilis, Pseudomonas, Streptomyces, etc.), it is necessary to operably link the desired enzyme encoding sequence to a functional prokaryotic promoter. Such promoters may be either constitutive or, more preferably, regulatable (i.e., inducible or derepressible). Examples of constitutive promoters include the int promoter of bacteriophage λ, and the bla promoter of the β-lactamase gene of pBR322, etc. Examples of inducible prokaryotic promoters include the major left and right promoters of bacteriophage λ - ($P_L$ and $P_R$), the trp, recA, lacZ, lacl, gal, and tac promoters of E. coli, the α-amylase (Ulmanen, I., et al., J. Bacteriol. 162:176-182 (1985)), the α-28-specific promoters of B. subtilis (Gilman, M.Z., et al., Gene 32:11-20 (1984)), the promoters of the bacteriophages of Bacillus (Gryczan, T.J., In: The Molecular Biology of the Bacilli, Academic Press, Inc., NY (1982)), and Streptomyces promoters (Ward, J.M., et al., Mol. Gen. Genet. 203:468-478 (1986)). Prokaryotic promoters are reviewed by Glick, B.R., (J. Ind. Microbiol. 1:277-282 (1987)); Cenatiempo, Y. (Biochimie 68:505-516 (1986)); and Gottesman, S. (Ann. Rev. Genet. 18:415-442 (1984)).

Proper expression in a prokaryotic cell also requires the presence of a ribosome binding site upstream from the gene-encoding sequence. Such ribosome binding sites are disclosed, for example, by Gold, L., et al. (Ann. Rev. Microbiol. 35:365-404 (1981)).

The desired enzyme encoding sequence and an operably linked promoter may be introduced into a recipient prokaryotic or eukaryotic cell either as a non-replicating DNA (or RNA) molecule, which may either be a linear molecule or, more preferably, a closed covalent circular molecule. Since such molecules are incapable of autonomous replication, the expression of the desired enzyme may occur through the transient expression of the introduced sequence. Alternatively, permanent expression may occur through the integration of the introduced sequence into the host chromosome.

In one embodiment, a vector is employed which is capable of integrating the desired gene sequences into the host cell chromosome. Cells which have stably integrated the introduced DNA into their chromosomes can be selected by also introducing one or more markers which allow for selection of host cells which contain the expression vector. The marker may complement an auxotrophy in the host (such as leu2, or ura3, which are common yeast auxotrophic markers), biocide resistance, e.g., antibiotics, or heavy metals, such as copper, or the like. The selectable marker gene can either be directly linked to the DNA gene sequences to be expressed, or introduced into the same cell by co-transfection.

In a preferred embodiment, the introduced sequence will be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector host between host cells of different species.

Any of a series of yeast gene expression systems can be utilized. Examples of such expression vectors include the yeast 2-micron circle, the expression plasmids YEP13, YCP and YRP, etc., or their derivatives. Such plasmids are well known in the art (Botstein, D., et al., Miami Wntr. Symp. 19:265-274 (1982); Broach, J.R., In: The Molecular Biology of the Yeast Saccharomyces: Life Cycle and Inheritance, Cold Spring Harbor Laboratory, Cold Spring Harbor, NY, p. 445-470 (1981); Broach, J.R., Cell 28:203-204)). YEP13 is the preferred vector of the present invention.

For a mammalian host, several possible vector systems are available for expression. One class of vectors utilize DNA elements which provide autonomously replicating extra-chromosomal plasmids, derived from animal viruses such as bovine papilloma virus, polyoma virus, adenovirus, or SV40 virus. A second class of vectors relies upon the integration of the desired gene sequences into the host chromosome. Cells which have stably integrated the introduced DNA into their chromosomes may be selected by also introducing one or more markers which allow selection of host cells which contain the expression vector. The marker may provide for prototropy to an auxotrophic host, biocide resistance, e.g., antibiotics, or heavy metals, such as copper or the like. The selectable marker gene can either be directly linked to the DNA sequences to be expressed, or introduced into the same cell by co-transformation. Additional elements may also be needed for optimal synthesis of mRNA. These elements may include splice signals, as well as transcription promoters, enhancers, and termination signals. The cDNA expression vectors incorporating such elements include those described by Okayama, H., Mol. Cell. Biol. 3:280 (1983), and others.

Preferred prokaryotic vectors include plasmids such as those capable of replication in E. coli (such as, for example, pBR322, ColE1, pSC101, pACYC 184, πVX. Such plasmids are, for example, disclosed by Maniatis, T., et al. (In: Molecular Cloning, A Laboratory Manual, Cold Spring Harbor Press, Cold Spring Harbor, NY (1982)). Bacillus plasmids include pC194, pC221, pT127, etc. Such plasmids are disclosed by Gryczan, T. (In: The Molecular Biology of the Bacilli, Academic Press, NY (1982), pp. 307-329). Suitable Streptomyces plasmids include pIJ101 (Kendall, K.J., et al., J. Bacteriol. 169:4177-4183 (1987)), and Streptomyces bacteriophages such as φC31 (Chater, K.F., et al., In: Sixth International Symposium on Actinomycetales Biology, Akademiai Kaido, Budapest, Hungary (1986), pp. 45-54). Pseudomonas plasmids are reviewed by John, J.F., et al. (Rev. Infect. Dis. 8:693-704 (1986)), and Izaki, K. (Jpn. J. Bacteriol. 33: 729-742 (1978)).

Once the vector or DNA sequence containing the constructs has been prepared for expression, the DNA constructs may be introduced into an appropriate host. Various techniques may be employed, such as protoplast fusion, calcium phosphate precipitation, electroporation or other conventional techniques. After the fusion, the cells are grown in media and screened for appropriate activities. Expression of the desired sequence results in the production of the substrate-specific aminopeptidase.

The substrate-specific aminopeptidase of the invention may be isolated and purified from the above-described recombinant molecules in accordance with conventional methods, such as extraction, precipitation, chromatography, affinity chromatography, electrophoresis, or the like.

## V. Uses of Substrate-Specific Aminopeptidase

Any of the substrate-specific aminopeptidases of the present invention, once produced and purified, can be used, for example, in a pharmaceutical, or manufacturing, environment to remove certain amino-terminal residues (consistent with each enzyme's substrate specificity as shown in Tables 2 and 3 below) from peptides and proteins for which such removal is desired in order to improve the stability, solubility, biologic activity, biologic half-life, etc. or to decrease the immunogenicity of the peptide or protein. It can, in addition, be used to cleave proteins, and polypeptides in accordance with its substrate specificities.

The substrate-specific aminopeptidase may be incubated with a desired substrate as a soluble enzyme, or may be immobilized, by means well known in the art, onto a solid support (such as, for example, glass, latex, plastic, cellulose, etc.)

Having now generally described this invention, the same will be better understood by reference to specific examples, which are included herein for purposes of illustration only, and are not intended to be limiting unless otherwise specified.

## EXAMPLE 1

## CELL GROWTH AND PREPARATION OF CRUDE EXTRACT

### Cell Growth and Storage

Yeast strain TD 71.8 (alpha, ino3, leu2, his3, lys2) was grown at 30 °C in 1 liter YPD medium, (1% yeast extract, 2% Bacto-peptone, 2% glucose) to $A_{600}$ ~18-20. The cells were harvested by centrifugation at 3,500 rpm for 10 min at 4 °C. The pellet (~25 g) was resuspended in 25 ml YPD medium containing 15% glycerol and stored at -70 °C.

## Preparation of Whole Cell Extracts

Frozen yeast cells (~500 g wet weight) were thawed in a 30 °C water bath and collected by centrifugation at 3,500 rpm for 10 minutes at room temperature. The pellet was resuspended in 1 liter of buffer S (1 M sorbitol, 50 mM Tris, pH 7.8, 10 mM $MgCl_2$, 3 mM DTT) containing 50 mg lyticase. This mixture was incubated at 30 °C for 2 hours with shaking to induce spheroplast formation. Spheroplasts were collected by centrifugation at 3,500 rpm for 10 minutes at 4 °C and washed by gentle resuspension in 1 liter of cold buffer S, collected by centrifugation at 3,500 rpm for 10 minutes and resuspended gently in 500 ml of buffer A (10 mM HEPES-$K^+$, pH 7.0, 1.5 mM $MgCl_2$, 10 mM KCl and 0.5 mM DTT). All the subsequent steps were carried out at 4 °C. The spheroplasts were lysed in this hypotonic buffer by 12-15 strokes with a tight-fitting pestle followed by 15-20 strokes with a loose-fitting pestle in a Dounce homogenizer and then the final KCl concentration was adjusted to 0.2 M by cold 2 M KCl solution. The lysate was stirred on ice for 30 minutes, and the debris was removed by centrifugation at 17,000 rpm for 30 minutes. Glycerol was added to 10% in all the buffers used in subsequent purification procedures to additionally stabilize the aminopeptidase.

The fractions were assayed for substrate-specific aminopeptidase activity as described above.

## EXAMPLE 2

## PURIFICATION OF THE SUBSTRATE-SPECIFIC AMINOPEPTIDASE

## Ammonium Sulfate Precipitation

The crude lysate was concentrated to 100 ml by the use of a PM-10 ultrafiltration membrane and dialyzed three times against 2 liters of buffer H (10 mM HEPES-$K^+$, pH 7.35, 1.5 mM $MgCl_2$, 0.5 mM DTT, 0.2% $NaN_3$, 10% glycerol) containing 0.05 M KCl. It was then loaded on a CM-Sepharose CL-6B column (2.5 x 45 cm) which had been connected to a DEAE-Sepharose CL-6B column (2.5 x 45 cm) such that the eluate from the CM-Sepharose CL-6B column was applied to the DEAE-Sepharose CL-6B column continually. Both columns have been equilibrated against the same buffer as that used for dialysis. These two connected columns were then eluted with 2 liters of buffer H containing 0.05 M KCl. The eluate of 800 ml from DEAE-Sepharose CL-6B column was collected. Solid ammonium sulfate (260.4 g) was added to this eluate to make a 50% saturated ammonium sulfate solution at 4 °C. It was stirred overnight at 4 °C and the precipitate was removed by centrifugation at 10,000 rpm for 30 min. Solid ammonium sulfate (188.4 g) was added to the supernatant to make an 80% saturated ammonium sulfate solution. It was stirred overnight and the precipitate was collected by centrifugation at 10,000 rpm for 30 min at 4 °C. This precipitate was redissolved in a minimum amount of buffer H containing 0.2 M KCl.

## Phenyl-Sepharose CL-4B Chromatography

The redissolved precipitate was dialyzed three times against 1 liter of buffer H (10 mM HEPES-$K^+$, pH 7.35, 0.5 mM DTT, 1.5 mM $MgCl_2$, 0.02% $NaN_3$, 10% glycerol) containing 0.2 M KCl. An equal volume of 80% saturated ammonium sulfate solution, 10 mM HEPES-$K^+$, pH 7.35, 10% glycerol, 0.02% $NaN_3$ was added to the solution containing aminopeptidase. This mixture was applied to a Phenyl-Sepharose CL-4B column (1.2 x 20 cm) equilibrated with 40% saturated ammonium sulfate solution, 10 mM HEPES-$K^+$, pH 7.35, 10% glycerol, 0.25 mM DTT, 0.1 M KCl, 0.02% $NaN_3$. The column was first eluted with 50 ml of the same buffer as that described above and then with a linear gradient of 40% (100 ml) to 0% (100 ml)

12

ammonium sulfate solution, 10 mM HEPES-K$^*$, pH 7.35, 10% glycerol, 0.25 mM DTT, 0.02% NaN$_3$, 100 mM KCl at 79 ml/hr. Fractions (3.95 ml) were collected. The fractions from 45 to 53 and 81 to 93 were pooled respectively. The aminopeptidase in the fractions 45 to 53 was designated as AP1; the one in the fractions 81- 93 was designated as AP122. The AP122 was further purified by a hydroxylapatite chromatography. AP1 was applied to a Sepharose CL-6B column.

Hydroxylapatite Chromatography

The pooled fractions (81 to 93) from the Phenyl-Sepharose CL-4B chromatography were concentrated to 5 ml by the use of a PM-10 ultrafiltration membrane. The concentration fraction was dialyzed three times against 1 liter of 0.01 M potassium buffer, 0.5 mM DTT, 1.5 mM MgCl$_2$, 0.2% NaN$_3$, 5 mM KCl, 10% glycerol, and applied to a hydroxylapatite column (2.5 x 20 cm) equilibrated with the same buffer as that used for dialysis. The column was eluted with a linear gradient of 0.01 M to 0.5 M potassium phosphate buffer 0.5 mM DTT, 0.2% NaN$_3$, 5 mM KCl, 10% glycerol at 19.1 ml/hr. Fractions (3.82 ml) were collected. The fractions containing aminopeptidase activity were pooled.

Sepharose-CL-4B Chromatography

The pooled fractions (45 to 53) from the Phenyl-Sepharose CL-4B chromatography were concentrated to a volume of 2 ml by using a PM-10 ultrafiltration membrane. It was dialyzed three times against 1 liter of buffer H containing a 0.2 M KCl and applied to a Sepharose CL-6B column (2.0 x 90 cm) equilibrated with the same buffer as that used for dialysis. The column was eluted with the buffer H containing 0.2 M KCl at 18.5 ml/hr. Fractions (3.7 ml) were collected. The fractions containing aminopeptidase activity were pooled.

The profiles of the fractions containing substrate-specific aminopeptidase activity are shown in Table 1.

Table 1

| Profile of Activity Containing Fractions | | | | |
|---|---|---|---|---|
| Column | Activity Fraction # | Data A$_{414}$ nm* | Protein Concentration 280 nm* | Average Conductivity of Active Fractions (ms) |
| AP1 | | | | |
| Phenyl-Sepharose CL-4B | 45-53 | 0.283 | 2.73 | 67.5 |
| Sepharose CL-6B | 53-59 | 0.583 | 0.673 | -- |
| AP122 | | | | |
| Phenyl-Sepharose CL-4B | 81-93 | 0.329 | 2.329 | 35 |
| Hydroxylapatite | 93-101 | 0.240 | 0.249 | 9.1 |

* Absorbance is average of pooled active fractions

EXAMPLE 3

ESTIMATION OF THE MOLECULAR WEIGHT OF AMINOPEPTIDASE AP1 BY SDS-POLYACRYLAMIDE GEL ELECTROPHORESIS

Electrophoresis was performed according to the method of Laemmli, U.K. (Nature 227:680-685 (1970)) using an 10% gel. The gel was stained with Coomassie blue, and the apparent molecular weight of the

EP 0 375 841 A1

denatured AP1 was calculated from the relative mobility of protein standards including (1) myosin (205,000), (2) E. coli betagalactosidase (116,000), (3) rabbit muscle phosphorylase (97,000), (4) bovine serum albumin (66,000) and (5) egg albumin (45,000), and (6) carbonic anhydrase (29,000). The apparent molecular weight of AP1 was approximately 81,000. The apparent molecular weight of AP122 has not been determined.

EXAMPLE 4

SUBSTRATE-SPECIFICITY

Thirty-six peptides were made to study the substrate specificity of the purified aminopeptidases of the present invention. Eighteen of the peptides had different N-terminal amino acid residues, and the remaining eighteen peptides had different amino acid residues at the penultimate amino-terminal position. The protein solution (10 $\mu$l) to be assayed was added to 90 $\mu$l of the solution containing 4 mM M-X-I-P-E-T or X-I-P-E-T, 10 mM HEPES-K$^+$, pH 7.35, 0.2 mM CoCl$_2$, 0.2 M KCl, 1.5 mM MgCl$_2$, 0.02% NaN$_3$, 30 $\mu$g of L-amino acid oxidase, 3 $\mu$g of horse radish peroxidase and 20 $\mu$g of o-dianisidine dihydrochloride. All the reactions were carried out at room temperature on a 96-well microtiter plate, and absorbance detected at 414 or 450 nm. Rapid color development indicates the presence of high peptidase activity on that specific peptide. The results of these experiments are shown in Tables 2 and 3. The identity of X is designated by the commonly used single letter abbreviations for the common amino acids: V = Val, A = Ala, R = Arg, D = Asp, C = Cys, E = Glu, Q-Gln, G = Gly, H = His, I = Ile, L = Leu, K-Lys, M = Met, F = Phe, P = Pro, S = Ser, T = Thr, and W = Trp. The numbers in Tables 2 and 3 represent qualitative estimates of activity: 0 (no activity); 1 (+/-); 2 (+); 3 (+ +/-); 4 (+ +); 5 (+ + +/-); and 6 (+ + +).

## Table 2
### Substrate-specificity: M-X-I-P-E-T

| Identity of X: | V | A | R | D | C | E | Q | G | H | I | L | K | M | F | P | S | T | W |
|---|---|---|---|---|---|---|---|---|---|---|---|---|---|---|---|---|---|---|
| ENZYME: AP1 | 1 | 2 | 4 | 0 | 2 | 0 | 0 | 4 | 4 | 3 | 3 | 2 | 1 | 4 | 0 | 0 | 0 | 0 |
| AP122 | 0 | 0 | 2 | 0 | 1 | 0 | 0 | 2 | 2 | 2 | 2 | 2 | 2 | 2 | 0 | 2 | 2 | 0 |

## Table 3
### Substrate-specificity: X-I-P-E-T

| Identity of X: | V | A | R | D | C | E | Q | G | H | I | L | K | M | F | P | S | T | W |
|---|---|---|---|---|---|---|---|---|---|---|---|---|---|---|---|---|---|---|
| ENZYME: AP1 | 0 | 0 | 2 | 0 | 0 | 0 | 1 | 0 | 0 | 0 | 6 | 0 | 6 | 6 | 0 | 0 | 0 | 1 |
| AP122 | 1 | 1 | 2 | 0 | 0 | 0 | 2 | 0 | 1 | 1 | 6 | 0 | 6 | 6 | 1 | 2 | 2 | 4 |

Although the foregoing refers to particular preferred embodiments, it will be understood that the present invention is not so limited. It will occur to those ordinarily skilled in the art that various modifications may be made to the disclosed embodiments and that such modifications are intended to be within the scope of the present invention.

**Claims**

1. The substantially purified substrate-specific aminopeptidase enzyme AP1 having an ability to cleave the peptide bond between an amino-terminal methionine residue, and a penultimate amino-terminal amino acid residue selected from the group consisting of: Val, Ala, Arg, Cys, Gly, His, Ile, Leu, Lys, Met and Phe, and having an ability to cleave the peptide bond between an amino-terminal residue X, and an adjacent Ile residue of a peptide, wherein X is an amino acid residue selected from the group consisting of: Arg, Gln, Leu, Met, Phe and Trp.

2. The substantially purified substrate-specific aminopeptidase enzyme AP122 having an ability to cleave the peptide bond between an amino-terminal methionine residue, and a penultimate amino-terminal amino acid residue selected from the group consisting of: Arg, Cys, Gly, His, Ile, Leu, Lys, Met, Phe, Ser or Thr, and having an ability to cleave the peptide bond between an amino-terminal residue X, and an adjacent Ile residue of a peptide, wherein X is an amino acid residue selected from the group consisting of: Val, Ala, Arg, Gln, His, Ile, Leu, Met, Phe, Pro, Ser, Thr and Trp.

3. A recombinant DNA molecule comprising a genetic sequence coding for the substrate-specific aminopeptidases of claims 1 or 2.

4. The substrate-specific aminopeptidases AP1 and AP 122 of claims 1 or 2 which are produced by the expression of a recombinant molecule.

5. A method for recovering the substantially purified substrate-specific aminopeptidase AP1 of claim 1 from a sample containing said aminopeptidase comprising the following steps:

(a) recovering crude substrate-specific aminopeptidase from a sample containing said aminopeptidase;

(b) subjecting said crude substrate-specific aminopeptidase from step (a) to serial ion exchange chromatography on CM-Sepharose and DEAE-Sepharose CL-6B; and

(c) recovering any substrate-specific aminopeptidase purified by ammonium sulfate precipitation from the eluate from said serial ion exchange chromatography, and, optionally:

(d) subjecting said recovered substrate-specific aminopeptidase of step (c) to Phenyl-Sepharose hydrophobic interaction chromatography; and

(e) recovering any substrate-specific aminopeptidase purified by said Phenyl-Sepharose hydrophobic interaction chromatography, and, further optionally:

(f) subjecting said recovered substrate-specific aminopeptidase of step (e) to Sepharose CL-6B chromatography; and

(g) recovering any substrate-specific aminopeptidase purified by said Sepharose CL-6B chromatography.

6. A method for recovering the substantially purified substrate-specific aminopeptidase AP122 of claim 2 from a sample containing said aminopeptidase comprising the following steps:

(a) recovering crude substrate-specific aminopeptidase from a sample containing said aminopeptidase;

(b) subjecting said crude substrate-specific aminopeptidase from step (a) to ion exchange chromatography; and

(c) recovering any substrate-specific aminopeptidase purified by ammonium sulfate precipitation from the eluate from said ion exchange chromatography, and, optionally:

(d) subjecting said recovered substrate-specific aminopeptidase of step (c) to Phenyl-Sepharose hydrophobic interaction chromatography; and

(e) recovering any substrate-specific aminopeptidase purified by said Phenyl-Sepharose hydrophobic interaction chromatography, and, further optionally:

(f) subjecting said recovered substrate-specific aminopeptidase of step (e) to hydroxylapatite chromatography; and

(g) recovering any substrate-specific aminopeptidase purified by said hydroxylapatite chromatography.

7. A method for removing an amino-terminal methionine residue from a peptide containing said amino-terminal residue, and containing as a penultimate amino-terminal amino acid residue, an amino acid selected from the group consisting of: Val, Ala, Arg, Cys, Gly, His, Ile, Lys, Met, Phe, and Leu, which method comprises incubating said peptide in the presence of the substrate-specific aminopeptidase AP1 to thereby remove said amino-terminal residue.

8. A method for removing an amino-terminal methionine residue from a peptide containing said amino-terminal residue, and containing as a penultimate amino-terminal amino acid residue, an amino acid selected from the group consisting of Arg, Cys, Gly, His, Ile, Lys, Met, Phe, Ser, Thr, and Leu, which method comprises incubating said peptide in the presence of the substrate-specific aminopeptidase AP122 to thereby remove said amino-terminal residue.

9. A method for removing an amino-terminal residue X from a peptide containing said terminal residue X, and an adjacent Ile residue, wherein X is selected from the group consisting of Arg, Gln, Leu, Met, Phe, Trp, which method comprises incubating said peptide in the presence of the substrate-specific aminopeptidase AP1 to thereby remove said amino-terminal residue.

10. A method for removing an amino-terminal residue X from a peptide containing said terminal residue X, and an adjacent Ile residue, wherein X is selected from the group consisting of Val, Ala, Arg, Gln, His, Ile, Leu, Met, Phe, Pro, Ser, Thr and Trp, which method comprises incubating said peptide in the presence of the substrate-specific aminopeptidase AP122 to thereby remove said amino-terminal residue.

11. The method of any of claims 7 to 10, wherein said substrate-specific aminopeptidase is immobilized to a solid support.

Claims for the following Contracting State: ES

1. A method for recovering the substantially purified substrate-specific aminopeptidase AP1 having an ability to cleave the peptide bond between an amino-terminal methionine residue, and a penultimate amino-terminal amino acid residue selected from the group consisting of: Val, Ala, Arg, Cys, Gly, His, Ile, Leu, Lys, Met and Phe, and having an ability to cleave the peptide bond between an amino-terminal residue X, and an adjacent Ile residue of a peptide, wherein X is an amino acid residue selected from the group consisting of: Arg, Gln, Leu, Met, Phe and Trp, from a sample containing said aminopeptidase, comprising the following steps:

(a) recovering crude substrate-specific aminopeptidase from a sample containing said aminopeptidase;

(b) subjecting said crude substrate-specific aminopeptidase from step (a) to serial ion exchange chromatography on CM-Sepharose and DEAE-Sepharose CL-6B; and

(c) recovering any substrate-specific aminopeptidase purified by ammonium sulfate precipitation from the eluate from said serial ion exchange chromatography, and, optionally,

(d) subjecting said recovered substrate-specific aminopeptidase of step (c) to Phenyl-Sepharose hydrophobic interaction chromatography; and

(e) recovering any substrate-specific aminopeptidase purified by said Phenyl-Sepharose hydrophobic interaction chromatography, and, further optionally:

(f) subjecting said recovered substrate-specific aminopeptidase of step (e) to Sepharose CL-6B chromatography; and

(g) recovering any substrate-specific aminopeptidase purified by said Sepharose CL-6B chromatography.

2. A method for recovering the substantially purified substrate-specific aminopeptidase AP122 having an ability to cleave the peptide bond between an amino-terminal methionine residue, and a penultimate amino-terminal amino acid residue selected from the group consisting of: Arg, Cys, Gly, His, Ile, Leu, Lys, Met, Phe, Ser or Thr, and having an ability to cleave the peptide bond between an amino-terminal residue X, and an adjacent Ile residue of a peptide, wherein X is an amino acid residue selected from the group consisting of: Val, Ala, Arg, Gln, His, Ile, Leu, Met, Phe, Pro, Ser, Thr and Trp,

(a) recovering crude substrate-specific aminopeptidase from a sample containing said aminopeptidase;

(b) subjecting said crude substrate-specific aminopeptidase from step (a) to ion exchange chromatography; and

(c) recovering any substrate-specific aminopeptidase purified by ammonium sulfate precipitation from the eluate from said ion exchange chromatography, and, optionally:

(d) subjecting said recovered substrate-specific aminopeptidase of step (c) to Phenyl-Sepharose hydrophobic interaction chromatography; and

(e) recovering any substrate-specific aminopeptidase purified by said Phenyl-Sepharose hydrophobic interaction chromatography, and, further optionally:

(f) subjecting said recovered substrate-specific aminopeptidase of step (e) to hydroxylapatite chromatography; and

(g) recovering any substrate-specific aminopeptidase purified by said hydroxylapatite chromatography.

3. A method for preparing the substrate-specific aminopeptidase AP1 or AP122, which comprises expressing in a host cell a recombinant DNA molecule encoding AP1 or AP122.

4. A method for removing an amino-terminal methionine residue from a peptide containing said amino-terminal residue, and containing as a penultimate amino-terminal amino acid residue, an amino acid selected from the group consisting of: Val, Ala, Arg, Cys, Gly, His, Ile, Lys, Met, Phe, and Leu, which method comprises incubating said peptide in the presence of the substrate-specific aminopeptidase AP1 to thereby remove said amino-terminal residue.

5. A method for removing an amino-terminal methionine residue from a peptide containing said amino-terminal residue, and containing as a penultimate amino-terminal amino acid residue, an amino acid selected from the group consisting of Arg, Cys, Gly, His, Ile, Lys, Met, Phe, Ser, Thr, and Leu, which method comprises incubating said peptide in the presence of the substrate-specific aminopeptidase AP122 to thereby remove said amino-terminal residue.

6. A method for removing an amino-terminal residue X from a peptide containing said terminal residue X, and an adjacent Ile residue, wherein X is selected from the group consisting of Arg, Gln, Leu, Met, Phe and Trp, which method comprises incubating said peptide in the presence of the substrate-specific aminopeptidase AP1 to thereby remove said amino-terminal residue.

7. A method for removing an amino-terminal residue X from a peptide containing said terminal residue X, and an adjacent Ile residue, wherein X is selected from the group consisting of Val, Ala, Arg, Gln, His, Ile, Leu, Met, Phe, Pro, Ser, Thr and Trp, which method comprises incubating said peptide in the presence of the substrate-specific aminopeptidase AP122 to thereby remove said amino-terminal residue.

8. The method of any one of claims 4 to 7, wherein said substrate-specific aminopeptidase is immobilized to a solid support.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | YEAST, vol. 1, 1985, pages 139-157, John Wiley & Sons Ltd; T. ACHSTETTER et al.: "Proteinases, Proteolysis and biological control in the yeast Saccharomyces cerevisiae" <br><br> --- | | C 12 N 9/60 <br> C 12 N 15/57 |
| A | CHEMICAL ABSTRACTS, vol. 81, no. 19, 11th November 1974, page 199, abstract no. 116439b, Columbus, Ohio, US; M.J. DESMAZEAUD et al.: "Revelation and partial characterization of various peptidases in Saccharomyces lactis", & ANN. BIOL. ANIM., BIOCHIM., BIOPHYS. 1974, 14(2), 327-41 <br><br> ----- | | |
| | | | **TECHNICAL FIELDS SEARCHED (Int. Cl.5)** <br><br> C 12 N |

The present search report has been drawn up for all claims

| Place of search | Date of completion of the search | Examiner |
|---|---|---|
| THE HAGUE | 20-01-1990 | PULAZZINI A.F.R. |